(19)

Europäisches Patentamt

European Patent Office

Office européen des brevets

(11) **EP 1 625 107 B1**

(12) **EUROPÄISCHE PATENTSCHRIFT**

(45) Veröffentlichungstag und Bekanntmachung des Hinweises auf die Patenterteilung:
**19.12.2007   Patentblatt 2007/51**

(21) Anmeldenummer: **04727886.6**

(22) Anmeldetag: **16.04.2004**

(51) Int Cl.:
***C07C 41/08*** *(2006.01)*       ***C07C 43/16*** *(2006.01)*

(86) Internationale Anmeldenummer:
**PCT/EP2004/004038**

(87) Internationale Veröffentlichungsnummer:
**WO 2004/096741 (11.11.2004 Gazette 2004/46)**

(54) **KONTINUIERLICHES VERFAHREN ZUR HERSTELLUNG VON METHYLVINYLETHER**

CONTINUOUS METHOD FOR PRODUCING METHYL VINYL ETHER

PROCEDE DE PRODUCTION EN CONTINU D'ETHER METHYLVINYLIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **30.04.2003   DE 10319452**

(43) Veröffentlichungstag der Anmeldung:
**15.02.2006   Patentblatt 2006/07**

(73) Patentinhaber: **BASF Aktiengesellschaft
67056 Ludwigshafen (DE)**

(72) Erfinder:
 • **KLASS, Katrin
   68159 Mannheim (DE)**
 • **BECKER, Heike
   68163 Mannheim (DE)**
 • **VOGELSANG, Regina
   67061 Ludwigshafen (DE)**
 • **HAUK, Alexander
   67071 Ludwigshafen (DE)**
 • **SIEGERT, Markus
   69126 Heidelberg (DE)**
 • **HENKELMANN, Jochem
   68165 Mannheim (DE)**

(56) Entgegenhaltungen:
**EP-A- 0 733 401          DE-B- 2 421 407
DE-C- 298 775**

 • **HANFORD W E ET AL: "ACETYLENE CHEMISTRY" INDUSTRIAL AND ENGINEERING CHEMISTRY, AMERICAN CHEMICAL SOCIETY, US, Bd. 40, Nr. 7, 1. Juli 1948 (1948-07-01), Seiten 1171-1177, XP000572476**

**Beschreibung**

Die vorliegende Erfindung betrifft ein Verfahren zur kontinuierlichen Herstellung von Methylvinylether durch Umsetzung von Methanol mit Ethin in der Flüssigphase in Gegenwart einer basischen Alkali- oder Erdalkalimetallverbindung bei einer Temperatur von 40 bis 300°C und einem Druck von 0,1 bis 5 MPa abs.

Vinylether stellen eine wichtige Verbindungsklasse mit breitem Einsatzgebiet dar. So finden sie unter anderem Verwendung als Monomerbausteine in Polymeren und Copolymeren, in Beschichtungen, Klebstoffen, Druckfarben sowie in strahlungshärtenden Lacken. Weitere Anwendungsgebiete sind die Herstellung von Zwischenprodukten, Geruchs- und Geschmacksstoffen sowie pharmazeutischen Produkten.

Die technische Herstellung von Vinylethern erfolgt in der Regel durch Umsetzung der entsprechenden Alkohole mit Ethin in Gegenwart basischer Katalysatoren (siehe Ull-mann's Encyclopedia of Industrial Chemistry, 6th edition, 2000 Electronic Release, Chapter "VINYL ETHERS - Production" und W. Reppe et al., Justus Liebigs Ann. Chem., 601 (1956), Seiten 135 bis 138). Die Vinylierung kann sowohl in der Flüssigphase als auch in der Gasphase durchgeführt werden. Bei der Vinylierung in der Gasphase werden basische Heterogenkatalysatoren, wie beispielsweise KOH auf Aktivkohle oder MgO oder CaO eingesetzt. In der Flüssigphase wird die stark exotherme Reaktion im Allgemeinen in Gegenwart von Alkalimetallhydroxid- oder Alkalimetallalkoxid-Katalysatoren durchgeführt.

DE-A 100 17 222 beschreibt ein Verfahren zur Herstellung von N-Alkenylethern durch Umsetzung eines Alkohols mit einem Alkin in Gegenwart einer basischen Alkalimetallverbindung und eines Mono- oder Diethers des 1,4-Butandiols als Cokatalysator. Die Vinylierung erfolgt diskontinuierlich, halbkontinuierlich oder kontinuierlich bei einer Temperatur von 100 bis 200°C, einem Ethin-Partialdruck von kleiner 5 MPa und einer Reaktionszeit von mehreren Stunden. Die genannten Beispiele betreffen die halbkontinuierliche Herstellung von tert.-Butyl-vinylether und Phenyl-vinylether in einem Autoklaven enthaltend das flüssige Reaktionsgemisch und eine darüberliegende Gasphase. Nachteilig an dem beschriebenen Verfahren ist die Ausbildung einer Gasphase, welche je nach Reaktionsführung eine entsprechend hohe Konzentration an nicht-umgesetzten Ethin enthalten kann und somit infolge der Zersetzungsneigung von Ethin zu einem sicherheitstechnischen Problem führen könnte.

SU 481 589 beschreibt ein Verfahren zur Herstellung von N-$C_1$- bis $C_5$-Alkyl-vinylethern durch Umsetzung des entsprechenden Alkanols mit Ethin in Gegenwart einer basischen Alkalimetallverbindung und des hochsiedenden Nebenprodukts der Vinylierungsreaktion als Lösungsmittel bei einer Temperatur von 100 bis 154°C und einem Druck von 0,7 bis 1,3 Atmosphären (etwa 0,07 bis 0,13 MPa abs). Nachteilig an dem beschriebenen Verfahren ist der Einsatz eines Lösungsmittels, welches eine weitere Komponente im Reaktionsgemisch darstellt und infolge dessen Volumenbedarfs zu einer Verminderung der Raum-Zeit-Ausbeute führt. Ferner ist die erforderliche Menge des einzusetzenden Lösungsmittels erst durch eine aufwändige und etwa 6 bis 12 Tage dauernde Vinylierung zugänglich. Des Weiteren von Nachteil ist die Ausbildung einer Gasphase, welche je nach Reaktionsführung eine entsprechend hohe Konzentration an nicht-umgesetzten Ethin enthalten kann und somit infolge der Zersetzungsneigung von Ethin zu einem sicherheitstechnischen Problem führen könnte.

DD-A 298 775 und DD-A 298 776 lehren Verfahren zur Herstellung von N-Alkylvinylethern durch Umsetzung eines Alkohols mit Ethin in Gegenwart basischer Alkalimetallverbindungen, eines Kronenethers beziehungsweise eines Polyethylenglykols als Cokatalysator und eines Lösungsmittels bei einer Temperatur von 20 bis 200°C und einem Druck von 0,05 bis 0,3 MPa abs, wobei das Ethin/Alkohol-Molverhältnis 1 bis 20 beträgt. Nach der Lehre der genannten Schriften ist die Reaktion unter Aufrechterhaltung einer geringen Alkoholkonzentration im Reaktionsgemisch zu führen, wobei größere Mengen an Alkohol im Reaktionsgemisch zu vermeiden sind. Bei der Herstellung niedrigsiedender Vinylether, wie beispielsweise Methyl-vinylether, werden diese zweckmäßigerweise kontinuierlich aus dem Reaktionsgemisch abdestilliert.

US 3,370,095 offenbart ein Verfahren zur kontinuierlichen oder batchweisen Herstellung niedermolekularer N-Alkyl-vinylether durch Umsetzung eines niedermolekularen Alkanols mit Ethin in Gegenwart einer basischen Alkalimetallverbindung und eines $C_{10}$- bis $C_{22}$-Alkanols oder dessen Vinylether als Lösungsmittel bei einer Temperatur von 160 bis 200°C und Atmosphärendruck, wobei das molare Verhältnis des niedermolekularen Alkanols zum Ethin 1 bis 2 beträgt. Unter der genannten erhöhten Temperatur und dem relativ niedrigen Druck wird der gebildete niedermolekularer N-Alkyl-vinylether zusammen mit dem überschüssigen niedermolekularen Alkanol und dem nicht-umgesetzten Ethin kontinuierlich aus der Flüssigphase verdampft und aus dem Reaktionsapparat ausgetragen.

Nachteilig an dem in DD-A 298 775, DD-A 298 776 und US 3,370,095 beschriebenen Verfahren ist der Einsatz eines Lösungsmittels, welches eine weitere Komponente im Reaktionsgemisch darstellt und infolge dessen Volumenbedarfs zu einer Verminderung der Raum-Zeit-Ausbeute führt. Ferner ist aufgrund der kontinuierlichen Abdestillation der niedrigsiedenden Alkyl-vinylether aus dem Reaktionsgemisch nur ein relativ geringer Reaktionsdruck möglich, welcher zu einer geringen Löslichkeit des Ethins in der Flüssigphase und somit zu einer nur geringen Reaktionsgeschwindigkeit führt. Zudem ist die Menge an zugeführten Ethin niedrig zu halten, da ansonsten eine Ethin-haltige Gasphase gebildet wird, welche infolge der Zersetzungsneigung von Ethin zu einem sicherheitstechnischen Problem führen könnte. Des Weiteren erfordert das in US 3,370,095 beschriebene Verfahren einen erhöhten Bedarf an Ethin, da der als Lö-

**EP 1 625 107 B1**

sungsmittel einzusetzende $C_{10}$- bis $C_{22}$-Alkanol im Laufe der Reaktion ebenfalls vinyliert wird.

EP-A 0 733 401 lehrt ein Verfahren zur diskontinuierlichen Umsetzung von Ethin mit einem Alkohol zum entsprechenden Vinylether in Gegenwart basischer Alkalimetallverbindungen in flüssiger Phase bei einer Temperatur von 0 bis 300˚C und einem Druck von 0,2 bis 3 MPa abs, bei dem man in Abwesenheit einer zusammenhängenden Gasphase Acetylen isobar bis zu einem Sättigungsgrad von 5 bis 100% in die flüssige Phase einleitet.

Nachteilig an den oben genannten halbkontinuierlichen oder diskontinuierlichen Verfahren sind die zeit-, arbeits- und energie-aufwendigen Arbeitsschritte durch Befüllen, Aufheizen, Aufpressen, Abkühlen, Entspannen und Entleeren des Reaktionsapparates, insbesondere bei der Herstellung von Mengen, welche mehrere Reaktionsansätze erfordern. Des Weiteren werden während der halbkontinuierlichen Synthesen ein großer Konzentrationsbereich bezüglich der Edukte- und Produktkonzentrationen durchlaufen, welcher zu einer umsatzabhängigen Reaktionsgeschwindigkeit führt und die Bildung unerwünschter Nebenprodukte fördern kann. Als Folge der genannten Nachteile resultiert eine geringere Selektivität und unter Berücksichtigung der erforderlichen Rüstzeiten auch eine deutlich geringere Raum/Zeit-Ausbeute als aufgrund der chemischen Reaktionsgeschwindigkeit möglich wäre.

DE-Az. 101 59 673.1 lehrt ein kontinuierliches Verfahren zur Herstellung von Vinylethern durch Umsetzung des entsprechenden Alkohols mit Ethin in der Flüssigphase in Gegenwart einer basischen Alkali- oder Erdalkalimetallverbindung bei 40 bis 300˚C und 0,11 bis 5 MPa abs, bei dem man einen Alkohol-Umsatz von ≥ 90% einstellt und in Abwesenheit einer zusammenhängenden Gasphase arbeitet.

Erfindungsgemäß wurde erkannt, dass die oben genannten Verfahren infolge des sehr niedrigen Siedepunkts von Methylvinylether von etwa 6˚C keine zufriedenstellende Lösung für ein sicherheitstechnisch unproblematisches und zugleich wirtschaftliches Verfahren bieten.

Demgemäß bestand die Aufgabe, ein Verfahren zur Herstellung von Methylvinylether durch Vinylierung von Methanol zu finden, was die oben genannten Nachteile nicht besitzt, einfach durchzuführen ist, sicherheitstechnisch insbesondere bezüglich der Handhabung von gasförmigen Ethin unproblematisch ist, eine hohe Selektivität zum Methylvinylether aufweist und eine insgesamt hohe Ausbeute und hohe Raum-Zeit-Ausbeute an Methylvinylether ermöglicht.

Demgemäß wurde ein Verfahren zur kontinuierlichen Herstellung von Methylvinylether durch Umsetzung von Methanol mit Ethin in der Flüssigphase in Gegenwart einer basischen Alkali- oder Erdalkalimetallverbindung bei einer Temperatur von 40 bis 300˚C und einem Druck von 0,1 bis 5 MPa abs gefunden, das dadurch gekennzeichnet ist, dass man die Umsetzung in Abwesenheit einer zusammenhängenden Gasphase und bei einer Methylyvinylether-Konzentration in der gesamten Flüssigphase von ≤ 30 Gew.-% durchführt.

Als zusammenhängende Gasphase sind dabei Gasräume innerhalb des Reaktionsraums zu verstehen, deren Größe über einzelne diskrete Blasen oder Bläschen hinausgeht. Zur Vermeidung einer zusammenhängenden Gasphase ist es wesentlich, die Umsetzung bei einer Methylvinylether-Konzentration in der gesamten Flüssigphase von ≤ 30 Gew.-% durchzuführen. Durch die genannte, relativ niedrige Konzentration des leichtflüchtigen Methylvinylethers wird der Dampfdruck des Gemisches deutlich erniedrigt, so dass dies der Bildung einer Gasphase entscheidend entgegenwirkt.

Bevorzugt führt man die Umsetzung bei einer Methylvinylether-Konzentration in der gesamten Flüssigphase von ≤ 25 Gew.-%, besonders bevorzugt ≤ 20 Gew.-%, ganz besonders bevorzugt ≤ 15 Gew.-% und insbesondere ≤ 10 Gew.-% durch. Die untere Grenze der Konzentration an Methylvinylether beträgt in der gesamten Flüssigphase im Allgemeinen ≥ 1 Gew.-%, bevorzugt ≥ 2 Gew.-% und besonders bevorzugt ≥ 5 Gew.-%. Der auf 100 Gew.-% fehlende Anteil in der Flüssigphase umfasst überschüssiges Methanol, die basische Alkali- oder Erdalkalimetallverbindung, gelöstes Ethin und eventuell gebildete Nebenprodukte sowie eventuell zugesetztes zusätzliches Lösungsmittel.

Als weitere Maßnahmen, welche neben des erfindungswesentlichen Merkmals der Methylvinylether-Konzentration in der gesamten Flüssigphase der Ausbildung einer zusammenhängenden Gasphase ergänzend entgegenwirken, sind eine gezielte Steuerung der Ethin-Konzentration in der gesamten Flüssigphase im Bereich der Ethin-Gaslöslichkeit in der Flüssigphase, eine intensive Durchmischung des Reaktionsgemisches und eine Entnahmemöglichkeit im Kopfbereich der Apparate und Leitungen genannt.

Im Allgemeinen gibt man das Ethin in einer Menge ≤ 100%, bevorzugt ≤ 95%, besonders bevorzugt ≤ 90% und ganz besonders bevorzugt ≤ 80% der maximalen Gaslöslichkeit in der Flüssigphase zu. Die untere Grenze der Ethin-Konzentration in der Flüssigphase ist im Zusammenhang mit der Vermeidung einer zusammenhängenden Gasphase unwesentlich.

Als Reaktoren können beim erfindungsgemäßen Verfahren prinzipiell die in der Fachliteratur beschriebenen Apparate für gas-flüssig-Umsetzungen eingesetzt werden. Um der Ausbildung einer zusammenhängenden Gasphase ergänzend entgegenzuwirken ist es vorteilhaft, eine intensive Durchmischung des Reaktionsgemisches zu bewirken. Diese kann beispielsweise durch einen effizienten Eintrag des Ethins in die Flüssigphase und/oder durch weitere Maßnahmen zur Durchmischung des Reaktionsgemisches, wie etwa durch intensives Rühren oder Erzeugen intensiver Strömungen erreicht werden. Eine intensive Durchmischung des Reaktionsgemisches trägt des Weiteren auch zu einer Erhöhung der Raum/Zeit-Ausbeute bei.

Die Umsetzung kann beim erfindungsgemäßen Verfahren in einem einzigen Reaktor oder in mehreren hinter-

3

einandergeschalteten Reaktoren, beispielsweise einer Reaktorkaskade durchgeführt werden. Als geeignete Reaktoren seien Rührkessel, Rührkesselkaskade, Strömungsrohre (bevorzugt mit Einbauten), Blasensäulen und Schlaufenreaktoren genannt. Um einen effizienten Eintrag zu erreichen, wird das Ethin beim Einsatz von Rührkesseln bevorzugt durch den Rührer und beim Einsatz von Strömungsrohren, Blasensäulen und Schlaufenreaktoren bevorzugt durch Düsen eingebracht.

Beim erfindungsgemäßen Verfahren besonders bevorzugt ist der Einsatz eines Strahlschlaufenreaktors. Durch das innere Einsteckrohr und die Zufuhr der Edukte und gegebenenfalls eines externen Kreislaufstroms über eine sogenannte Strahldüse wird ein innerer Kreislaufstrom erzeugt, welcher eine besonders intensive Durchmischung bewirkt. Um einen ausreichend großen Impulseintrag durch die Strahldüse zu gewährleisten wird der Strahlschlaufenreaktor bevorzugt mit einem externen Kreislauf betrieben. Hierbei wird kontinuierlich Reaktionsgemisch entnommen und über eine Pumpe und gegebenenfalls einem Wärmetauscher zur Strahldüse zurückgeführt. Die Strahldüse kann am Boden oder am Kopf des Reaktors angebracht sein, wobei sich im letztgenannten Fall vorzugsweise ein konzentrisches Einsteckrohr entlang der Strömungsrichtung des eingebrachten Flüssigkeitsstroms mittig im Reaktor befindet. Die Anordnung der Strahldüse im höchsten Punkt des Reaktors besitzt den Vorteil, dass bei Stillstand der Flüssigkeitsumwälzung keine Flüssigkeit in den gasführenden Teil der Strahldüse zurücksteigen kann. Entsprechend befindet sich beim Einsatz einer kopfseitigen Strahldüse die Entnahme des Kreislaufstroms am Boden des Reaktors. Die Verwendung einer selbstansaugenden Strahldüse stellt eine zusätzliche, integrierte Sicher heitseinrichtung dar, da bei Ausfall der Umwälzpumpe und damit des Flüssigkeitstreibstrahls kein Gas mehr angesaugt werden kann.

Um eine theoretisch mögliche Ansammlung von Gasblasen am Kopf des Reaktors sicher zu verhindern, wird das für die anschließende Aufarbeitung und Isolierung des Methylvinylethers zu entnehmende Reaktionsgemisch bevorzugt an der höchsten Stelle des Reaktors entnommen.

Um das restliche, in Lösung befindliche Ethin ebenfalls mit dem Methanol umzusetzen, ist ein Verfahren bevorzugt, bei dem man die Umsetzung in einem Hauptreaktor unter Zufuhr der Ausgangsverbindungen Methanol und Ethin und einem oder mehreren Nachreaktoren ohne weitere Zufuhr der Ausgangsverbindungen durchführt. Als Nachreaktoren können beispielsweise Strömungsrohre oder Verweilzeitbehälter eingesetzt werden. Um eine Rückvermischung zu vermeiden, werden bevorzugt kaskadierte Nachreaktoren eingesetzt. Im Allgemeinen weisen die Nachreaktoren ein kleineres Volumen als der Hauptreaktor auf. Zur Vervollständigung der Reaktion im Nachreaktor beziehungsweise in den Nachreaktoren genügt im Allgemeinen eine Verweilzeit von 5 Minuten bis 1 Stunde. Somit ist ein Ethin-Umsatz von bis zu 100% möglich.

Die Umsetzung wird beim erfindungsgemäßen Verfahren bei einer Temperatur von 40 bis 300°C, bevorzugt von 60 und 230°C, besonders bevorzugt von 70 bis 200°C und ganz besonders bevorzugt von 80 bis 150°C durchgeführt. Sie wird bei einem Druck von 0,1 bis 5 MPa abs, bevorzugt von 0,15 bis 3 MPa abs und besonders bevorzugt von 0,5 bis 3 MPa abs durchgeführt.

Methanol und Ethin werden im Allgemeinen entsprechend ihres Verbrauchs zugeführt. Die zugeführten Mengen entsprechen daher in etwa den stöchiometrisch erforderlichen Mengen, korrigiert um mögliche Einflüsse wie etwa durch Austrag nicht-umgesetzter Edukte oder durch Nebenproduktbildung.

Beim erfindungsgemäßen Verfahren setzt man die basische Alkali- oder Erdalkalimetallverbindung im Allgemeinen in einer Menge von 0,05 bis 15 Gew.-%, bevorzugt von 0,1 bis 10 Gew.-% und besonders bevorzugt von 0,1 bis 5 Gew.-%, bezogen auf das Reaktionsgemisch, ein.

Als basische Alkali- und Erdalkalimetallverbindungen setzt man beim erfindungsgemäßen Verfahren im Allgemeinen die Oxide, Hydroxide und Alkoholate, wie beispielsweise die $C_1$- bis $C_4$-Alkanolate wie Methanolat, Ethanolat, 1-Propanolat, 2-Propanolat, 1-Butanolat, 2-Butanolat, 2-Methyl-1-propanolat und 2-Methyl-2-propanolat des Lithiums, Natriums, Kaliums, Cäsiums, Magnesiums und Kalziums ein. Als Kationen bevorzugt sind Natrium und Kalium, insbesondere Kalium. Als Anionen bevorzugt sind Hydroxid und Alkoholate. Die Alkali- und Erdalkalimetallalkoholate sind beispielsweise durch Umsetzung der Alkali- oder Erdalkalimetalle, deren Oxide oder Hydroxide mit dem entsprechenden Alkohol unter Entfernung der gebildeten Nebenprodukte Wasserstoff beziehungsweise Wasser erhältlich. Beim Einsatz der Oxide und Hydroxide erhitzt man diese im Allgemeinen zusammen dem entsprechenden Alkohol unter Verdampfung des gebildeten Wassers. Auch der Einsatz von Mischungen verschiedener Alkali- und Erdalkalimetallverbindungen ist möglich.

Werden die Oxide oder Hydroxide eingesetzt, so stehen diese mit dem Methanol unter Bildung von Wasser und Methanolat im chemischen Gleichgewicht. Dies führt, bezogen auf die eingesetzte Menge an Oxid beziehungsweise Hydroxid zu einem relativ geringen Anteil an chemisch wirksamen Methanolat, was zum Ausgleich den Einsatz einer deutlich größeren Menge an Oxid beziehungsweise Hydroxid bedarf.

In diesem Zusammenhang wurde ferner gefunden, dass beim Einsatz von Alkoholaten eine geringere Nebenproduktbildung auftritt und eine zusammenhängende Gasphase erst bei noch größeren Methylvinylether-Konzentrationen auftritt. Ersteres führt zu einem Vorteil in der nachfolgenden Aufarbeitung, letzteres zu einer höheren Sicherheitsreserve.

Besonders bevorzugt ist daher der Einsatz von Alkoholaten, ganz besonders bevorzugt von Methanolat und insbesondere von Kaliummethanolat.

Beim erfindungsgemäßen Verfahren ist es möglich und gegebenenfalls vorteilhaft, sogenannte Cokatalysatoren einzusetzen. Sie können gegebenenfalls eine Verringerung der Nebenproduktbildung bewirken. Der Einsatz von Cokatalysatoren ist allgemein bekannt und beispielsweise in DE-A 3 215 093, US 5,665,889, DE-A 100 17 222, WO 01/46139 und WO 01/46141 beschrieben, worauf explizit Bezug genommen wird. Als bevorzugte Beispiele möglicher Cokatalysatoren seien Polyoxyalkylenverbindungen (z.B. Polyoxyethylen oder Polyoxypropylen) oder Divinylverbindungen von Diolen (z.B. 1,2-Divinyloxyethan oder 1,4-Divinyloxybutan) genannt. Sie werden in der Regel in einer Menge von 2 bis 30 Gew.-% eingesetzt. Je nach Art und Menge des eingesetzten Cokatalysators kann dieser gegebenenfalls auch als Lösungsmittel angesehen werden.

Beim erfindungsgemäßen Verfahren ist es prinzipiell auch möglich Lösungsmittel einzusetzen. Geeignete Lösungsmittel zeichnen sich dadurch aus, dass sich in ihnen sowohl Methylvinylether als auch Methanol und die basische Alkali- und Erdalkalimetallverbindung lösen, sie chemisch nicht mit diesen Verbindungen reagieren, d.h. vor allem keine aciden Zentren besitzen, welche die basische Gruppen abfangen würden, und sie sich vom gebildeten Methylvinylether ohne großen Aufwand, bevorzugt destillativ, entfernen lassen. Als Beispiele geeigneter Lösungsmittel seien die dipolar aprotischen Lösungsmittel N-Methylpyrrolidon, Tetrahydrofuran und die Dialkylether von Glykolen, Di-, Oligo- oder Polyglykolen genannt. Das erfindungsgemäße Verfahren wird bevorzugt ohne zusätzliches Lösungsmittel durchgeführt.

Der Methanol-Umsatz bei einem Reaktordurchgang, welcher definitionsgemäß auf die dem Reaktor zugeführte Menge an Methanol und auf die aus dem Reaktor abgeführte Menge an Methanol bezogen wird, ist in erster Linie davon abhängig, ob das Verfahren mit einem zusätzlichen Lösungsmittel durchgeführt wird oder nicht. Wird das Verfahren mit einem zusätzlichen Lösungsmittel durchgeführt, so kann dieses gegebenenfalls den Hauptteil des flüssigen Reaktionsgemisches darstellen, um die erforderliche Konzentration an Methylvinylether sicherzustellen. In diesem Fall kann der Methanol-Umsatz sogar bis zu 100% betragen.

In der bevorzugten Ausführungsform ohne Einsatz eines zusätzlichen Lösungsmittels besteht der Großteil der Flüssigphase aus nicht-umgesetzten Methanol. Daher ergibt sich rechnerisch ein geringer Methanol-Umsatz bei einem Reaktordurchgang, welcher im Allgemeinen im Bereich von etwa 5% bis etwa 25% liegt.

Das aus dem Reaktor entnommene und den Methylvinylether, Methanol, die basische Alkali- oder Erdalkalimetallverbindung, Ethin und gegebenenfalls Nebenprodukte und ein eventuell zugesetztes Lösungsmittel enthaltende Reaktionsgemisch wird im Allgemeinen anschließend aufgearbeitet. Die Aufarbeitung erfolgt dabei in der Regel destillativ. Aufgrund des relativ niedrigen Siedepunktes des Methylvinylethers von etwa 6˚C wird dieser üblicherweise in einer Kolonne und bevorzugt in einem Dünnschichtverdampfer über Kopf abgetrennt und in einer nachgeschalteten Kolonne von Leichtsiedern wie hauptsächlich nicht-umgesetztem Ethin abgetrennt und daraus als Sumpfprodukt gewonnen. Das Sumpfprodukt der ersten Kolonne enthält im Wesentlichen Methanol, die basische Alkali- oder Erdalkalimetallverbindung und gegebenenfalls höher siedende Nebenprodukte sowie eventuell zugesetztes Lösungsmittel und wird bevorzugt zum Vinylierungsreaktor zurückgeführt. Um eine Aufpegelung an Nebenprodukten zu vermeiden und eine gleichbleibend hohe Aktivität der basischen Alkali- oder Erdalkalimetallverbindung zu gewährleisten wird üblicherweise ein kontinuierlicher Strom als Purge-Strom entnommen und eine frische Lösung der basischen Alkali- oder Erdalkalimetallverbindung kontinuierlich zugegeben.

Besonders bevorzugt ist ein erfindungsgemäßes Verfahren, bei dem man

(a) die Umsetzung unter Zufuhr der Ausgangsverbindungen Methanol und Ethin in einem Reaktor durchführt,

(b) das durch die Umsetzung erhaltene Reaktionsgemisch entspannt,

(c) das entspannte Reaktionsgemisch in einem Dünnschichtverdampfer in einen Methylvinylether enthaltenden Kopfstrom und einen Methanol und die basische Alkali- oder Erdalkalimetallverbindung enthaltenden Sumpfstrom trennt, und

(d) mindestens einen Teil des Alkali- oder Erdalkalimetallverbindung enthaltenden Sumpfstroms zum Reaktor zurückführt.

Die bevorzugte Anlage zur Herstellung von Methylvinylether nach dem erfindungsgemäßen Verfahren umfasst einen Strahlschlaufenreaktor mit externem Kreislauf, einen kaskadierten Nachreaktor, einen Dünnschichtverdampfer zur Abtrennung des gebildeten Methylvinylethers und eine Pumpe zur Rückführung der von Methylvinylether abgetrennten methanolischen Lösung. Beim erfindungsgemäßen Verfahren werden Methanol, Kaliummethanolat und Ethin dem Strahlschlaufenreaktor kontinuierlich zugeführt. Die Zufuhr erfolgt über eine kopfseitige Strahldüse, welche in Verbindung mit dem konzentrischen Einsteckrohr zu einer intensiven Durchmischung des Reaktionsgemisches führt. Das für den externen Kreislauf zu entnehmende Reaktionsgemisch wird im unteren Teil des Strahlschlaufenreaktors entnommen, über eine Kreislaufpumpe und einen Wärmetauscher geführt und zur Strahldüse geleitet. Das aufzuarbeitende Reaktionsgemisch wird am Kopf des Strahlschlaufenreaktors entnommen und in den kaskadierten Nachreaktor geleitet. Das den Nachreaktor verlassende Reaktionsgemisch wird auf etwa Atmosphärendruck entspannt und abgekühlt und dem Dünnschichtverdampfer zur Auftrennung zugeführt. Diesem wird über Sumpf eine Lösung, enthaltend das nicht-umgesetzte Methanol und Kaliummethanolat, entnommen und über eine Rückführungspumpe zum externen Reaktorkreislauf zurückgeführt. Um eine Aufpegelung an Schwersiedern zu verhindern wurde ein kleiner Strom als Purge-Strom ausgeschleust. Die über den Kopf des Dünnschichtverdampfers entnommene Gasphase wird in einer weiteren Kolonne in Methylvinylether und einen Ethin-haltigen Abgasstrom getrennt.

Das erfindungsgemäße Verfahren ermöglicht die kontinuierliche Herstellung von Methylvinylether durch Vinylierung von Methanol, welche einfach durchzuführen ist, sicherheitstechnisch insbesondere bezüglich der Handhabung von gasförmigen Ethin unproblematisch ist, eine hohe Selektivität zum Methylvinylether aufweist und eine insgesamt hohe Ausbeute und hohe Raum-Zeit-Ausbeute an Methylvinylether ermöglicht.

Beispiele

Definitionen

Die in den Beispielen angegebenen Werte für Ethin-Umsatz, Methanol-Umsatz und Raum-Zeit-Ausbeute sind durch folgende Gleichungen definiert:

$$\text{Ethin-Umsatz} \quad = \quad \frac{\dot{n}_{C2H2,in} - \dot{n}_{C2H2,out}}{\dot{n}_{C2H2,in}}$$

$$\text{Methanol-Umsatz} \quad = \quad \frac{\dot{n}_{MeOH,in} - \dot{n}_{MeOH,out}}{\dot{n}_{MeOH,in}}$$

$$\text{Raum-Zeit-Ausbeute} \quad = \quad \frac{\dot{m}_{MVE}}{V_{reactor}}$$

| | |
|---|---|
| $\dot{m}_{MVE}$ | Massenstrom an produziertem Methylvinylether [g/h] |
| $V_{reactor}$ | Volumen des Hauptreaktors [L] |
| $\dot{n}_{C2H2,in}$ | Stoffmengenstrom an Ethin, welcher in den ersten Reaktor geleitet wird, unabhängig von dessen Herkunft, das heißt inklusive Rückführung [mol/h] |
| $\dot{n}_{C2H2,out}$ | Stoffmengenstrom an Ethin, welcher aus dem letzten Reaktor ausgangsseitig entnommen wird [mol/h] |
| $\dot{n}_{MeOH,in}$ | Stoffmengenstrom an Methanol, welcher in den ersten Reaktor geleitet wird, unabhängig von dessen Herkunft, das heißt inklusive Rückführung [mol/h] |
| $\dot{n}_{MeOH,out}$ | Stoffmengenstrom an Methanol, welcher aus dem letzten Reaktor ausgangsseitig entnommen wird [mol/h] |

Versuchsanlage

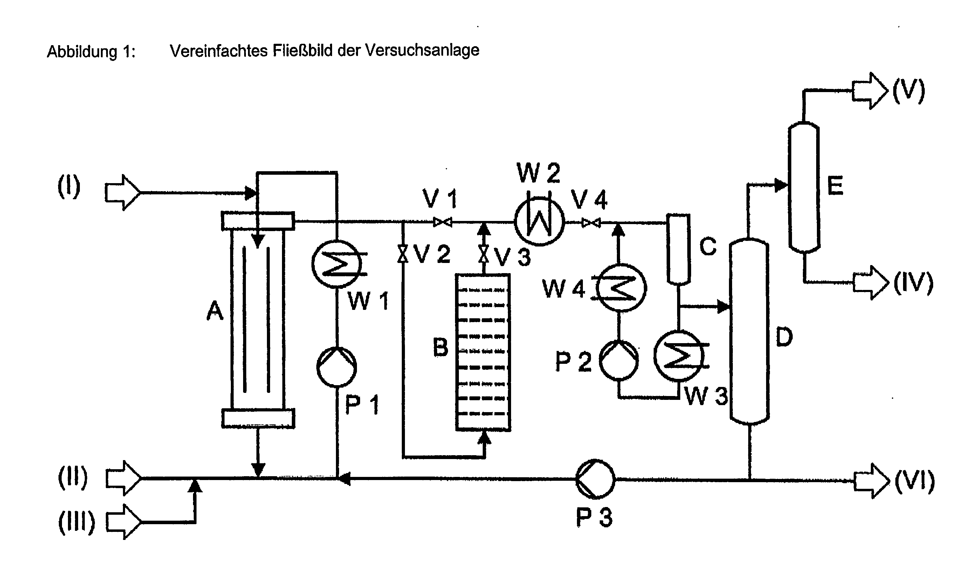

Das vereinfachte Verfahrensfließbild der kontinuierlich arbeitenden Versuchsanlage ist in Abbildung 1 dargestellt. Methanol (II) und eine Lösung aus einer basischen Kaliumverbindung (Kaliumhydroxid in Beispiel 1 und Kaliummethylat in Beispiel 2) in Methanol (III) werden mit dem Rückführungsaustrag des Reaktors A und dem Rückführungsstrom der basischen Kaliumverbindung in Methanol zusammengeführt und über die Pumpe P1 und dem Wärmetauscher W1 zum Kopf des Reaktors A geführt. Dort wird in diesen Zufuhrstrom Ethin (1) eingespeist und dieser durch eine Düse in den Reaktor A eingeleitet. Als Reaktor A wurde ein mantelbeheizter Strahlschlaufenreaktor mit einem Einsteckrohr und einem Gesamtvolumen von 1,6 L eingesetzt. In diesem wird durch den Impuls des über die Düse eingeleiteten Zufuhrstroms ein interner Kreislaufstrom erzeugt. Der Reaktor A ist während des Betriebs soweit gefüllt, dass sich keine zusammenhängende Gasphase ausbilden kann. Über die bereits beschriebene Pumpe P1 und den Wärmetauscher W1 wird zudem ein externer Kreislaufstrom erzeugt. Die Entnahme des aufzuarbeitenden Reaktionsgemisches erfolgt am Kopf des Reaktors A. Je nach Versuchsdurchführung war es möglich, das Reaktionsgemisch zur Erhöhung des Ethin-Umsatzes, das heißt zur Abreaktion des restlichen gelösten Ethins, durch einen Nachreaktor B zu leiten. Bei diesem handelte es sich um einen kaskadierten Rohrreaktor mit einem Volumen von 1,4 L, der zur Verhinderung einer Gasblasenbildung mit Blenden versehen ist. Wurde das Reaktionsgemisch durch den Nachreaktor B geleitet, so waren die Ventile V2 und V3 geöffnet und das Ventil V1 geschlossen. Wurde der Nachreaktor B umgangen, so waren die Ventile V2 und V3 geschlossen und das Ventil V1 geöffnet. Das Reaktionsgemisch wurde über den Wärmetauscher W2 gekühlt und durch das Entspannungsventil V4 auf etwa Atmosphärendruck entspannt. Anschließend wurde das Reaktionsgemisch in einem Quenschkreislauf, welcher einen Behälter C, einen Wärmetauscher W3, eine Kreislaufpumpe P2 und einen weiteren Wärmetauscher W4 umfasste, weiter abgekühlt. Das abgekühlte Reaktionsgemisch wurde aus dem Quenschkreislauf entnommen und zur Abtrennung des nicht-umgesetzten Methanols und der basischen Kaliumverbindung vom Methylvinylether und gegebenenfalls vorhandenen Ethin in einen Dünnschichtverdampfer D geleitet. Die über Sumpf entnommene Lösung, enthaltend das nicht-umgesetzte Methanol und die basische Kaliumverbindung, wurde über die Pumpe P3 zum externen Reaktorkreislauf zurückgeführt. Um eine Aufpegelung an Schwersiedern zu verhindern wurde ein kleiner Strom als Purge-Strom (VI) ausgeschleust. Die über den Kopf des Dünnschichtverdampfers D entnommene Gasphase wurde in einer weiteren Kolonne E in Methylvinylether (IV) und einen Ethin-haltigen Abgasstrom (V) getrennt.

Versuchsdurchführung

Zum Anfahren der Versuchsanlage wurde der Strahlschlaufenreaktor A mit Methanol und der basischen Kaliumverbindung in entsprechender Menge gefüllt, der externe Kreislauf in Betrieb genommen und das System auf die gewünschte Temperatur gebracht. Anschließend wurde auch der Nachreaktor B, sofern dieser in dem Versuchslauf eingesetzt wurde, der Quenschkreislauf, der Dünnschichtverdampfer D und die Rückführung des Methanols und der basischen Kaliumverbindung in Betrieb genommen. Durch Zufuhr von Ethin (1) und Methanol wurde anschließend mit der Vinylierungsreaktion begonnen und die gewünschten Parameter, wie beispielsweise Zulaufmengen, Druck, Temperatur, Kreislauf- und Rücklaufmengen, in der Versuchsanlage eingestellt.

Die Versuchsparameter und Ergebnisse der Beispiele 1 und 2 sind in Tabelle 1 dargestellt.

Die Beispiele zeigen, dass nach dem erfindungsgemäßen Verfahren auch bei einer Methylvinylether-Konzentration von ≤ 30 Gew.-% und der Umsetzung in Abwesenheit einer zusammenhängenden Gasphase eine hohe Selektivität zum Methylvinylether und eine hohe Ausbeute und hohe Raum-Zeit-Ausbeute an Methylvinylether erreicht wird.

Tabelle 1: Versuchsparameter und Ergebnisse der Beispiele 1 und 2

|  | Beispiel 1 | Beispiel 2 |
|---|---|---|
| basische Kaliumverbindung | Kaliumhydroxid (KOH) als 25 Gew.-%ige Lösung in Methanol | Kaliummethanolat (KOMe) als 32 Gew.-%ige Lösung in Methanol |
| Zuläufe | 50 g/h Ethin 85 g/h Methanol + 3,66 g/h KOH 500 g/h Rückführungsstrom | 55 g/h Ethin 70 g/h Methanol + 0,14 g/h KOMe 680 g/h Rückführungsstrom |
| Strahlschlaufenreaktor A: Temperatur Druck | 110˚C 2,0 MPa abs | 110˚C 2,0 MPa abs |
| Nachreaktor B: Temperatur Druck | nicht in Betrieb | 110˚C 2,0 MPa abs |
| Methylvinylether-Konzentration in der Flüssigphase vor dem Entspannungsventil V4 | 20 Gew.-% | 30 Gew.-% |
| Zusammenhängende Gasphase | nein | nein |
| Umsätze | 95% Ethin-Umsatz 14 bis 16% Methanol-Umsatz | > 99% Ethin-Umsatz 10 bis 12% Methanol-Umsatz |
| Raum-Zeit-Ausbeite (bezogen auf Strahlschlaufenreaktor A) | 70 g/Lh | 80 g/Lh |

**Patentansprüche**

1. Verfahren zur kontinuierlichen Herstellung von Methylvinylether durch Umsetzung von Methanol mit Ethin in der Flüssigphase in Gegenwart einer basischen Alkali- oder Erdalkalimetallverbindung bei einer Temperatur von 40 bis 300˚C und einem Druck von 0,1 bis 5 MPa abs, **dadurch gekennzeichnet, dass** man die Umsetzung in Abwesenheit einer zusammenhängenden Gasphase und bei einer Methylvinylether-Konzentration in der gesamten Flüssigphase von ≤ 30 Gew.-% durchführt.

2. Verfahren nach Anspruch 1, **dadurch gekennzeichnet, dass** man die Umsetzung bei einer Methylvinylether-Konzentration in der gesamten Flüssigphase von ≤ 20 Gew.-% durchführt.

3. Verfahren nach den Ansprüchen 1 bis 2, **dadurch gekennzeichnet, dass** man das Ethin in einer Menge ≤ 100% der maximalen Gaslöslichkeit in der Flüssigphase zugibt.

4. Verfahren nach den Ansprüchen 1 bis 3, **dadurch gekennzeichnet, dass** man die Umsetzung in einem Strahlschlaufenreaktor durchführt.

5. Verfahren nach den Ansprüchen 1 bis 4, **dadurch gekennzeichnet, dass** man die Umsetzung in einem Hauptreaktor unter Zufuhr der Ausgangsverbindungen Methanol und Ethin und einem oder mehreren Nachreaktoren ohne weitere Zufuhr der Ausgangsverbindungen durchführt.

6. Verfahren nach den Ansprüchen 1 bis 5, **dadurch gekennzeichnet, dass** man die Umsetzung bei einer Temperatur von 80 bis 150˚C durchführt.

7. Verfahren nach den Ansprüchen 1 bis 6, **dadurch gekennzeichnet, dass** man die Umsetzung bei einem Druck von 0,5 bis 3 MPa abs durchführt.

8. Verfahren nach den Ansprüchen 1 bis 7, **dadurch gekennzeichnet, dass** man die basische Alkali- oder Erdalkalimetallverbindung in der Flüssigphase in einer Konzentration von 0,05 bis 15 Gew.-% einsetzt.

9. Verfahren nach den Ansprüchen 1 bis 8, **dadurch gekennzeichnet, dass** man als basische Alkali- oder Erdalkalimetallverbindung Kaliummethanolat einsetzt.

10. Verfahren nach den Ansprüchen 1 bis 9, **dadurch gekennzeichnet, dass** man

(a) die Umsetzung unter Zufuhr der Ausgangsverbindungen Methanol und Ethin in einem Reaktor durchführt,
(b) das durch die Umsetzung erhaltene Reaktionsgemisch entspannt,
(c) das entspannte Reaktionsgemisch in einem Dünnschichtverdampfer in einen Methylvinylether enthaltenden Kopfstrom und einen Methanol und die basische Alkali- oder Erdalkalimetallverbindung enthaltenden Sumpfstrom trennt, und
(d) mindestens einen Teil des Alkali- oder Erdalkalimetallverbindung enthaltenden Sumpfstroms zum Reaktor zurückführt.

**Claims**

1. A process for continuously preparing methyl vinyl ether by reacting methanol with ethyne in the liquid phase in the presence of a basic alkali metal or alkaline earth metal compound at a temperature of from 40 to 300˚C and a pressure of from 0.1 to 5 MPa abs, which comprises carrying out the reaction in the absence of a continuous gas phase and at a methyl vinyl ether concentration in the entire liquid phase of ≤ 30% by weight.

2. A process as claimed in claim 1, wherein the reaction is carried out at a methyl vinyl ether concentration in the entire liquid phase of ≤ 20% by weight.

3. A process as claimed in either of claims 1 or 2, wherein the ethyne is added in an amount of ≤ 100% of the maximum gas solubility in the liquid phase.

4. A process as claimed in any of claims 1 to 3, wherein the reaction is carried out in a jet loop reactor.

# EP 1 625 107 B1

5. A process as claimed in any of claims 1 to 4, wherein the reaction is carried out in a main reactor while feeding the starting compounds, methanol and ethyne, and one or more postreactors without any further feed of the starting compounds.

6. A process as claimed in any of claims 1 to 5, wherein the reaction is carried out at a temperature of from 80 to 150˚C.

7. A process as claimed in any of claims 1 to 6, wherein the reaction is carried out at a pressure of from 0.5 to 3 MPa abs .

8. A process as claimed in any of claims 1 to 7, wherein the basic alkali metal or alkaline earth metal compound is used in the liquid phase in a concentration of from 0.05 to 15% by weight.

9. A process as claimed in any of claims 1 to 8, wherein the basic alkali metal or alkaline earth metal compound used is potassium methoxide.

10. A process as claimed in any of claims 1 to 9, wherein

(a) the reaction is carried out in a reactor while feeding the starting compounds, methanol and ethyne,
(b) the reaction mixture obtained by the reaction is decompressed,
(c) the decompressed reaction mixture is separated in a thin-film evaporator into a top stream comprising methyl vinyl ether and a bottom stream comprising methanol and the basic alkali metal or alkaline earth metal compound, and
(d) at least a portion of the bottom stream comprising the alkali metal or alkaline earth metal compound is recycled to the reactor.

## Revendications

1. Procédé de préparation en continu d'éther méthylvinylique par réaction de méthanol avec de d'acétylène, en phase liquide, en présence d'un composé basique de métal alcalin ou alcalino-terreux, à une température de 40 à 300˚C et une pression de 0,1 à 5 MPa abs., **caractérisé en ce que** l'on entreprend la réaction en l'absence d'une phase gazeuse cohérente et avec une concentration d'éther méthylvinylique dans l'ensemble de la phase liquide ≤ 30% en poids.

2. Procédé suivant la revendication 1, **caractérisé en ce que** l'on entreprend la réaction avec une concentration d'éther méthylvinylique dans l'ensemble de la phase liquide ≤ 20% en poids.

3. Procédé suivant les revendications 1 à 2, **caractérisé en ce que** l'on ajoute l'acétylène dans la phase liquide en une quantité ≤ 100% de la solubilité maximale du gaz dans la phase liquide.

4. Procédé suivant les revendications 1 à 3, **caractérisé en ce que** l'on entreprend la réaction dans un réacteur à jet et à boucle de circulation.

5. Procédé suivant les revendications 1 à 4, **caractérisé en ce que** l'on entreprend la réaction dans un réacteur principal avec amenée des composés de départ méthanol et acétylène et un ou plusieurs post-réacteurs sans autre amenée des composés de départ.

6. Procédé suivant les revendications 1 à 5, **caractérisé en ce que** l'on entreprend la réaction à une température de 80 à 150˚C.

7. Procédé suivant les revendications 1 à 6,
**caractérisé en ce que** l'on entreprend la réaction à une pression de 0,5 à 3 MPa.

8. Procédé suivant les revendications 1 à 7, **caractérisé en ce que** l'on met en oeuvre le composé basique de métal alcalin ou alcalino-terreux dans la phase liquide en une concentration de 0,05 à 15% en poids .

9. Procédé suivant les revendications 1 à 8, **caractérisé en ce que** l'on met en oeuvre en tant que composé basique de métal alcalin ou, alcalino-terreux du méthanolate de potassium.

**10.** Procédé suivant les revendications 1 à 9, **caractérisé en ce que** :

(a) on entreprend la réaction avec amenée des composés de départ méthanol et acétylène dans un réacteur,

(b) on détend le mélange réactionnel obtenu par la réaction,

(c) on sépare le mélange réactionnel détendu, dans un évaporateur à couche mince, en un courant de tête contenant de l'éther méthylvinylique et un courant de fond contenant du méthanol et le composé basique de métal alcalin ou alcalino-terreux, et

(d) on renvoie dans le réacteur au moins une partie du courant de fond contenant le composé basique de métal alcalin ou alcalino-terreux.

Abbildung 1: Vereinfachtes Fließbild der Versuchsanlage

EP 1 625 107 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10017222 A
- SU 481589
- DD 298775 A
- DD 298776 A
- US 3370095 A
- EP 0733401 A
- DE 10159673
- DE 3215093 A
- US 5665889 A
- WO 0146139 A
- WO 0146141 A

**In der Beschreibung aufgeführte Nicht-Patentliteratur**

- VINYL ETHERS - Production. *Ull-mann's Encyclopedia of Industrial Chemistry,* 2000
- **W. REPPE et al.** *Justus Liebigs Ann. Chem.,* 1956, vol. 601, 135-138